# EUROPEAN PATENT SPECIFICATION

(11) **EP 0 452 363 B1**
(45) Date of publication and mention of the grant of the patent: **20.07.1994**
(21) Application number: 90901441.7
(22) Date of filing: 21.12.1989
(51) Int. Cl.: C07C 19/08, C07C 17/00, B01J 27/125

(54) **ALUMINUM FLUORIDE CATALYST AND USE THEREOF IN A CHLOROFLUORINATION PROCESS FOR PREPARING 1,1-DICHLORO-1,2,2,2-TETRAFLUOROETHANE**
ALUMINIUMFLUORID KATALYSATOR UND SEINE VERWENDUNG IN EINEM CHLOROFLUORIERUNGSVERFAHREN ZUR HERSTELLUNG VON 1,1-DICHLOR-1,2,2,2-TETRAFLUORETHAN
FLUORURE D'ALUMINIUM POUR LA CATALYSE ET SON EMPLOI DANS UN PROCEDE DE CHLOROFLUORATION POUR LA PREPARATION DE 1,1-DICHLORO-1,2,2,2-TETRAFLUOROETHANE

(30) Priority: 28.12.1988 US 291100
(43) Date of publication of application: 23.10.1991
(73) Proprietor: E.I. DU PONT DE NEMOURS AND COMPANY, Wilmington Delaware 19898 (US)
(72) Inventor: MANZER, Leo, Ernest, Wilmington, DE 19803 (US); TEBBE, Frederick, Nye, Hockessin, DE 19707 (US)
(74) Representative: Jones, Alan John
(86) International application number: PCT/US89/05738
(87) International publication number: WO 90/07482

(56) References cited:
- EP-A- 0 056 548
- WO-A-89/11467
- GB-A- 1 578 933

## Description

This invention relates to catalysts and their use for the manufacture of 1,1-dichloro-1,2,2,2-tetra-fluoroethane (i.e. "CFC-114a"), and more particularly to aluminum fluoride catalysts and use thereof for preparing CFC-114a.

### BACKGROUND OF THE INVENTION

Fluorinated aluminas are well known as fluorination or chlorofluorination catalysts. For example the use of both aluminum fluoride and aluminum fluoride containing iron, chromium and nickel for fluorination or chlorofluorination reactions is described in U.S. Patent No. 3,650,987. The fluorided alumina is often prepared by the addition of HF to Al₂O₃. The products from these reactions contain large amounts of the symmetrical isomers of various chlorofluorocarbons. The addition of metal dopants increases the amount of symmetrical isomers.

German (DDR) Patent Specification 117,580 discloses a process for the preparation of asymmetrical fluorochlorocarbon compounds of the C₂ series (e.g. CFC-114a) by the reaction of tetrachloroethylene, chlorine and hydrogen fluoride over a metal doped aluminum fluoride catalyst. In the example of this patent with the highest amount of asymmetrical products, the 1,1-dichloro-1,2,2,2-tetrafluoroethane (CFC-114a)/1,2-dichloro-1,1,2,2-tetra-fluoroethane (CFC-114) ratio is about 11.5; and the 1,1,1-trichloro-2,2,2-trifluoroethane (CFC-113a)/1,1,2-trichloro-1,2,2-trifluoroethane (CFC-113) ratio is about 8.0. However 57.4% of the product is chloropentafluoroethane (CFC-115), which is generally considered an undesirable byproduct in CFC-114a manufacturing processes. In another example where 6.1% of the product is CFC-115, the ratios of CFC-114a/CFC-114 and CFC-113a/CFC-113 are 7.3 and 3.8 respectively.

European Patent Application 317,981 discloses a process for isomerizing CFC-113 to CFC-113a followed by fluorination with HF to produce CFC-114a. In the example of this patent with the highest ratio of CFC-114a/CFC-114 (52.7) the ratio of CFC-113a/CFC-113 is 1.1. In other examples the CFC-114a/CFC-114 ratio varied from 45.3 to 5.5 and the CFC-113a/CFC-113 ratio from 6.2 to 1.1.

Japanese Kokai 1-172347 discloses a process for the preparation of CFC-114a by first disproportionating CFC-114 to CFC-113a followed by reaction with HF. In the examples the CFC-114a/CFC-114 ratios vary from 9.0 to 16.7 and the CFC-113a/CFC-113 ratios from 0.1 to >36. In the high 113a/113 ratio example the yield of 114a is only 15.0%. In the other examples the yields of 114a varied from 43.0% to 51.0%.

It is also known in the art (GB 1,578,933) that both CClF₂CClF₂ (CFC-114) and CF₃CCl₂F (CFC-114a) may be hydrogenated over a Pd catalyst to CHF₂CHF₂ (HFC-134) and CF₃CH₂F (HFC-134a) respectively. The latter compound (HFC-134a) is considered a refrigerant suitable for replacing CCl₂F₂ since it does not significantly contribute to stratospheric ozone depletion while CCl₂F₂ is suspected of being a major contributor.

Accordingly, there is continued interest in developing economic and efficient processes for preparing 1,1-dichloro-1,2,2,2-tetrafluoroethane.

### SUMMARY OF THE INVENTION

In accordance with this invention, a process is provided for preparing 1,1-dichloro-1,2,2,2-tetrafluoroethane by chlorofluorination. The process comprises the step of contacting a gaseous mixture comprising at least one tetrahaloethylene having the formula C₂CCl₄₋ₓFₓ, wherein x is an integer from zero to 3, and both Cl₂ and HF with a catalyst at an elevated temperature, said catalyst consisting of a high purity aluminum fluoride prepared by the reaction of aluminum hydroxide and HF. The 1,1-dichloro-1,2,2,2-tetrafluoroethane prepared by the process may be hydrodechlorinated to produce 2-chloro-1,1,1,2-tetrafluoroethane and/or tetrafluoroethane. A catalyst is also provided in accordance with this invention which consists of a high purity aluminum fluoride prepared by the reaction of aluminum hydroxide with HF.

### DETAILED DESCRIPTION OF THE INVENTION

A process for preparing 1,1-dichloro-1,2,2,2-tetrafluoroethane by chlorofluorination is provided in accordance with this invention and comprises the step of contacting a gaseous mixture comprising at least one tetrahaloethylene and both Cl₂ and HF with a catalyst at an elevated temperature, said catalyst consisting of a high purity aluminum fluoride prepared by the reaction of aluminum hydroxide and HF. Tetrahaloethylenes useful in this invention include those having the formula C₂Cl₄₋ₓFₓ, wherein x is zero or an integer from 1 to 3. Examples of tetrahaloethylenes useful for this process include CCl₂=CCl₂, CClF=CCl₂, CClF=CClF, CF₂=CCl₂, and CF₂=CClF, and mixtures of these. Tetrachloroethylene is preferred.

The reaction of the tetrahaloethylene with HF and Cl₂ in the presence of the catalyst of the instant invention is conducted at an elevated temperature. Suitable temperatures are generally within the range of about 300°C to 450°C. Preferably the temperature is within the range of about 300°C to 400°C, and most preferably is within the range of about 350°C to 375°C. Contact times can influence the yield of the reaction to some extent. Preferably the temperature and contact time are balanced to achieve a yield of at least about 95% total of CFC-114a and recyclable by-products (i.e. by-products having less than 5 fluorine atoms per molecule); and preferably the aluminum fluoride purity of the catalyst is sufficient to provide a molar ratio of 1,1-dichloro-1,2,2,2-tetrafluoroethane to 1-2-dichloro-1,1,2,2-tetrafluoroethanes (i.e. of CFC-114a to CFC-114) of at least about 45 and most preferably sufficient to provide a molar ratio of CFC-114a to CFC-114 of at least about 47.5. A contact time within the range of about 5 to 100 seconds is typical. Preferably the contact time is within the range of about 10 to 90 seconds, and most preferably is within the range of about 15 to 60 seconds.

The HF in the gaseous mixture is preferably at least the stoichiometric amount needed to produce CFC-114a. The molar ratio of the HF to the tetrahaloethylene is typically within the range of about 1:1 to 20:1. Preferably the ratio of HF to the tetrahaloethylene is within the range of about 3:1 to 10:1, and most preferably is within the range of about 4:1 to 7:1.

Preferably Cl₂ is provided in the gaseous mixture in at least the amount needed to produce CFC-114a. The molar ratio of Cl₂ to the tetrahaloethylene is preferably within the range of about 1:1 to 2:1, and most preferably is about 1:1.

The desired product of the present invention can be separated by a usual method such as fractional distillation. By-products having less than five fluorine atoms per molecule such as C₂Cl₅F, C₂Cl₄F₂, C₂F₃Cl₃, and CClF₂CClF₂ are considered recyclable, and may advantageously be further contacted with the catalyst used in this invention.

The reaction of the tetrahaloethylene with HF and chlorine may be conducted in any suitable reactor, including fixed and fluidized bed reactors which are charged with the catalyst. The reaction vessel should be constructed from materials which are resistant to the corrosive effects of HF and Cl₂ such as Hastelloy® nickel alloy and Inconel® nickel alloy. Optionally, before the catalyst is contacted by the tetrahaloethylenes, it may be pretreated with gaseous HF.

Pressure is not critical. Atmospheric and superatmospheric pressures are the most convenient and are therefore preferred.

1,1-dichloro-1,2,2,2-tetrafluoroethane produced by this invention has utility as a solvent and as an intermediate for the preparation of 2-chloro-1,1,1,2-tetrafluoroethane and/or 1,1,1,2-tetrafluoroethane. Indeed an improved process for producing 1,1,1,2-tetrafluoroethane and/or 2-chloro-1,1,2,2-tetrafluoroethane by hydrodechlorinating 1,1-dichloro-1,2,2,2-tetrafluoroethane is provided in accordance with this invention. The improvement comprises the step of preparing CFC-114a by chlorofluorination using tetrahaloethylenes and the catalyst of this invention as described above.

Catalysts are provided in accordance with this invention which consist of an aluminum fluoride prepared by the reaction of aluminum hydroxide with HF.

The catalyst of this invention may be suitably prepared by reacting aqueous HF (e.g. 48% solution) with aluminum hydroxide. Suitable aluminum hydroxide may be prepared by the hydrolysis of AlR₃, where each R is independently selected from C₁ to C₆ alkyl groups. For example, a preparation of high purity Al(OH)₃ prepared by the hydrolysis of Al(CH₂CH₃)₃ is described by F. N. Tebbe et al., J. Am. Ceram. Soc., 71 [4], C-204 - C-206, (1988). Suitable aluminum hydroxide may also be prepared by hydrolysis of Al(OR)₃, where each R is independently selected from C₁ to C₆ alkyl groups. For example, high purity Al(OH)₃ may be prepared by the hydrolysis of Al(OCH(CH₃)CH₂CH₃)₃.

A particularly useful catalyst consisting of an aluminum fluoride is prepared by dissolving aluminum hydroxide in aqueous HF; evaporating the resulting solution to obtain a residue of an aluminum fluoride; and heating said residue to produce a dried solid. The catalysts of this invention are also useful for catalyzing other reactions such as the chlorofluorination of CF₃CCl₃, CF₃CHClF and/or CF₃CH₃ to CFC-114a, isomerization of CClF₂-CClF₂ to CCl₂F-CF₃, isomerization of CHF₂-CHF₂ to CH₂F-CF₃, fluorination of CF₃-CHCl₂ to CF₃-CHClF and fluorination of CHCl=CCl₂ to CClF₂CH₂Cl and CF₃CH₂Cl.

Practice of the invention will become further apparent from the following non-limiting examples.

### EXAMPLE I

Aluminum sec-butoxide (Alfa, 95%), about 300 g, was placed in an open dish and allowed to hydrolyze in air over two days. The aluminum hydroxide produced was a powder and clusters of powdery solids. In a polyethylene tray a portion of the aluminum hydroxide (39 g) was dissolved in 48% aqueous hydrofluoric acid (100 mL). The volatiles were evaporated at ambient temperature over three days in a fume hood. The resulting white residue was dried at 110°C for 48 hours. The solid was then heated in air at a rate of 5°C/min to 500°C, and held at this temperature for three hours. After cooling, the solid (30.3 g) was crushed and passed through a sieve to yield fines (12.8 g) and a fraction of granules 12x20 mesh (1.4 x 0.83 mm) in size (17.5 g). Analysis showed 31.5% Al and 227 ppm (0.0227%) of metal ion impurities.

### EXAMPLE II

A reactor (a 0.5 inch (1.3 cm) ID, 12 inch (30.5 cm) long Inconel® nickel alloy pipe) was charged with the aluminum fluoride catalyst composition (15.7 g, 25 mL) prepared as described in Example I above. The charged reactor was placed in a sand bath. The bath was gradually heated to 250°C while nitrogen gas at a flow rate of 50cc/minute was passed through the reactor to remove traces of water. HF and nitrogen gas (1:4 molar ratio) were then passed through the reactor. An exotherm of about 10°C, which travelled down the reactor, was observed. The temperature was gradually raised to 450°C, the nitrogen flow decreased with time until neat HF was passed through the reactor. The HF flow was stopped after no further exotherm was recorded. HF/CCl₂=CCl₂/Cl₂ in a 5/1/1 molar ratio was then passed over the catalyst at 375°C and a 30 second contact time. The reactor effluent was sampled on-line with a Hewlett-Packard 5890 gas chromatograph using a 20 foot (6.1 m) long, 1/8" (0.32 cm) diameter, column containing Krytox® perfluorinated polyether on an inert support and a helium flow of 35 cc/minute. Gas chromatographic conditions were 70°C for 3 minutes followed by temperature programming to 180°C at a rate of 6°C/minute. Analysis showed the following to be present (area percent): 25.4% CCl₂=CCl₂, 0.2% CCl₃CCl₂F, 0.5% CCl₂FCCl₂F/CClF₂CCl₃, 1.6% CCl₂FCClF₂ (CFC-113), 9.4% CF₃CCl₃ (CFC-113a), 1.2% CClF₂CClF₂ (CFC-114), 58.8% CF₃CCl₂F (CFC-114a), 1.0% CClF₂CF₃ (CFC-115), 0.1% CClF₂CHCl₂, and 0.2% CCl₂=CClF.

The yield to useful products including both CFC-114a and recyclable by-products, is greater than 98% and the molar ratio of CFC-114a/CFC-114 is 49.

## Claims (Claims for the following Contracting State(s): AT, BE, CH, DE, FR, GB, IT, LI, LU, NL, SE)

1. A process for preparing 1,1-dichloro-1,2,2,2-tetrafluoroethane by chlorofluorination comprising the step of contacting a gaseous mixture comprising at least one tetrahaloethylene having the formula C₂Cl₄₋ₓFₓ, wherein x is an integer from zero to 3, and both Cl₂ and HF with a catalyst at a temperature within the range of 300°C to 450°C, said catalyst consisting of a high purity aluminum fluoride prepared by the reaction of aluminum hydroxide and HF.

2. The process of Claim 1 wherein the at least one tetrahaloethylene is CCl₂=CCl₂.

3. The process of Claim 1 wherein by-products having less than five fluorine atoms per molecule are recycled.

4. The process of Claim 1 wherein the molar ratio of said HF to said tetrahaloethylene is within the range of 1:1 to 20:1; and wherein the molar ratio of said Cl₂ to said tetrahaloethylene is within the range of 1:1 to 2:1.

5. The process of Claim 1 wherein the contact times are within the range of 5 to 100 seconds.

6. The process of Claim 1 wherein before the catalyst is contacted by said at least one tetrahaloethylene it is pretreated with HF gas.

7. An improved process for producing 2-chloro-1,1,1,2-tetrafluoroethane and/or 1,1,1,2-tetrafluoroethane by hydrodechlorinating 1,1-dichloro-1,2,2,2-tetrafluoroethane, the improvement comprising the step of preparing said 1,1-dichloro-1,2,2,2-tetrafluoroethane by contacting a gaseous mixture comprising at least one tetrahaloethylene having the formula C₂Cl₄₋ₓFₓ, wherein x is zero or an integer from 1 to 3, and both Cl₂ and HF with a catalyst at a temperature within the range of 300°C to 450°C, said catalyst consisting of of a high purity aluminum fluoride prepared by the reaction of aluminum hydroxide and HF.

8. A catalyst consisting of a high purity aluminum fluoride prepared by the reaction of aluminum hydroxide with HF.

9. The catalyst of Claim 8 prepraed by dissolving aluminum hydroxide in aqueous HF; evaporating the resulting solution to obtain a residue of an aluminum fluoride; and heating said residue to produce a dried solid.

10. The catalyst of Claim 8 wherein said aluminum hydroxide is prepared by the hydrolysis of AlR₃ or the hydrolysis of Al(OR)₃ where each R is independently selected from C₁ to C₆ alkyl groups.

## Claims (Claims for the following Contracting State(s): ES)

1. A process for preparing 1,1-dichloro-1,2,2,2-tetrafluoroethane by chlorofluorination comprising the step of contacting a gaseous mixture comprising at least one tetrahaloethylene having the formula C₂Cl₄₋ₓFₓ, wherein x is an integer from zero to 3, and both Cl₂ and HF with a catalyst at a temperature within the range of 300°C to 450°C, said catalyst consisting of a high purity aluminum fluoride prepared by the reaction of aluminum hydroxide and HF.

2. The process of Claim 1 wherein the at least one tetrahaloethylene is CCl₂=CCl₂.

3. The process of Claim 1 wherein by-products having less than five fluorine atoms per molecule are recycled.

4. The process of Claim 1 wherein the molar ratio of said HF to said tetrahaloethylene is within the range of 1:1 to 20:1, and wherein the molar ratio of said Cl₂ to said tetrahaloethylene is within the range of 1:1 to 2:1.

5. The process of claim 1 wherein the contact times are within the range of 5 to 100 seconds.

6. The process of Claim 1 wherein before the catalyst is contacted by said at least one tetrahaloethylene it is pretreated with HF gas.

7. An improved process for producing 2-chloro-1,1,1,2-tetrafluoroethane and/or 1,1,1,2-tetrafluoroethane by hydrodechlorinating 1,1-dichloro-1,2,2,2-tetrafluoroethane, the improvement comprising the step of preparing said 1,1-dichloro-1,2,2,2-tetrafluoroethane by contacting a gaseous mixture comprising at least one tetrahaloethylene having the formula C₂Cl₄₋ₓFₓ, wherein x is zero or an integer from 1 to 3, and both Cl₂ and HF with a catalyst at a temperature within the range of 300°C to 450°C, said catalyst consisting of a high purity aluminum fluoride prepared by the reaction of aluminum hydroxide and HF.

8. The process of claim 7 wherein the catalyst is prepared by dissolving aluminum hydroxide in aqueous HF; evaporating the resulting solution to obtain a residue of an aluminum fluoride; and heating said residue to produce a dried solid.

9. The process of Claim 7 wherein said aluminum hydroxide is prepared by the hydrolysis of A1R₃ or the hydrolysis of A1(OR)₃ where each R is independently selected from C₁ to C₆ alkyl groups.

## Patentansprüche (Patentansprüche für folgende(n) Vertragsstaat(en): AT, BE, CH, DE, FR, GB, IT, LI, LU, NL, SE)

1. Verfahren zur Herstellung von 1,1-Dichlor-1,2,2,2-tetrafluorethan durch Chlorfluorierung, umfassend die Stufe des in-Kontakt-Bringens einer gasförmigen Mischung, die wenigstens ein Tetrahalogenethylen der Formel C₂Cl₄₋ₓFₓ enthält, worin x eine ganze Zahl von null bis 3 ist, und sowohl Cl₂ als auch HF, mit einem Katalysator bei einer Temperatur im Bereich von 300 °C bis 450 °C, worin der Katalysator aus einem Aluminiumfluorid hoher Reinheit besteht, das durch die Reaktion von Aluminiumhydroxid mit HF hergestellt wird.

2. Verfahren gemäß Anspruch 1, worin das wenigstens eine Tetrahalogenethylen CCl₂ = CCl₂ ist.

3. Verfahren gemäß Anspruch 1, worin Nebenprodukte, die weniger als fünf Fluoratome pro Molekül aufweisen, im Kreislauf rückgeführt werden.

4. Verfahren gemäß Anspruch 1, worin das Stoffmengenverhältnis des HF zu dem Tetrahalogenethylen im Bereich von 1:1 bis 20:1 liegt und worin das Stoffmengenverhältnis des Cl₂ zu dem Tetrahalogenethylen im Bereich von 1:1 bis 2:1 liegt.

5. Verfahren gemäß Anspruch 1, worin die Kontaktzeiten im Bereich von 5 bis 100 Sekunden liegen.

6. Verfahren gemäß Anspruch 1, worin vor dem in-Kontakt-Bringen des Katalysators mit dem wenigstens einen Tetrahalogenethylen, dieser mit HF-Gas vorbehandelt wird.

7. Verbessertes Verfahren zur Herstellung von 2-Chlor-1,1,1,2-tetrafluorethan und/oder 1,1,1,2-Tetrafluorethan durch Hydrodechlorierung von 1,1-Dichlor-1,2,2,2-tetrafluorethan, wobei die Verbesserung die Stufe der Herstellung von 1,1-Dichlor-1,2,2,2-tetrafluorethan durch inKontakt-Bringen einer gasförmigen Mischung, die wenigstens ein Tetrahalogenethylen der Formel C₂Cl₄₋ₓFₓ enthält, worin x null oder eine ganze Zahl von 1 bis 3 ist, und sowohl Cl₂ als auch HF, mit einem Katalysator bei einer Temperatur im Bereich von 300 °C bis 450 °C, worin der Katalysator aus einem Aluminiumfluorid hoher Reinheit besteht, das durch die Reaktion von Aluminiumhydroxid mit HF hergestellt wird, umfaßt.

8. Katalysator, bestehend aus einem Aluminiumfluorid von hoher Reinheit, das durch Reaktion von Aluminiumhydroxid mit HF hergestellt wird.

9. Katalysator gemäß Anspruch 8, der durch Ausflösen von Aluminiumhydroxid in wäßrigem HF, Eindampfen der erhaltenen Lösung, um einen Rückstand eines Aluminiumfluorids zu erhalten, und Erwärmen des Rückstandes unter Bildung eines getrockneten Feststoffs hergestellt wird.

10. Katalysator gemäß Anspruch 8, worin das Aluminiumhydroxid durch die Hydrolyse von AlR₃ oder die Hydrolyse von Al(OR)₃, worin jedes R unabhängig voneinander aus C₁- bis C₆-Alkylgruppen ausgewählt ist, hergestellt wird.

## Patentansprüche (Patentansprüche für folgende(n) Vertragsstaat(en): ES)

1. Verfahren zur Herstellung von 1,1-Dichlor-1,2,2,2-tetrafluorethan durch Chlorfluorierung, umfassend die Stufe des in-Kontakt-Bringens einer gasförmigen Mischung, die wenigstens ein Tetrahalogenethylen der Formel C₂Cl₄₋ₓFₓ enthält, worin x eine ganze Zahl von null bis 3 ist, und sowohl Cl₂ als auch HF, mit einer Katalysator-Zusammensetzung bei einer Temperatur im Bereich von 300 °C bis 450 °C, worin die Katalysator-Zusammensetzung aus einem Aluminiumfluorid hoher Reinheit besteht, das durch die Reaktion von Aluminiumhydroxid mit HF hergestellt wird.

2. Verfahren gemäß Anspruch 1, worin das wenigstens eine Tetrahalogenethylen CCl₂ = CCl₂ ist.

3. Verfahren gemäß Anspruch 1, worin Nebenprodukte, die weniger als fünf Fluoratome pro Molekül aufweisen, im Kreislauf rückgeführt werden.

4. Verfahren gemäß Anspruch 1, worin das Stoffmengenverhältnis des HF zu dem Tetrahalogenethylen im Bereich von 1:1 bis 20:1 liegt und worin das Stoffmengenverhältnis des Cl₂ zu dem Tetrahalogenethylen im Bereich von 1:1 bis 2:1 liegt.

5. Verfahren gemäß Anspruch 1, worin die Kontaktzeiten im Bereich von 5 bis 100 Sekunden liegen.

6. Verfahren gemäß Anspruch 1, worin vor dem in-Kontakt-Bringen des Katalysators mit dem wenigstens einen Tetrahalogenethylen, dieser mit HF-Gas vorbehandelt wird.

7. Verbessertes Verfahren zur Herstellung von 2-Chlor-1,1,1,2-tetrafluorethan und/oder 1,1,1,2-Tetrafluorethan durch Hydrodechlorierung von 1,1-Dichlor-1,2,2,2-tetrafluororethan, wobei die Verbesserung die Stufe der Herstellung von 1,1-Dichlor-1,2,2,2-tetrafluorethan durch in-Kontakt-Bringen einer gasförmigen Mischung, die wenigstens ein Tetrahalogenethylen der Formel C₂Cl₄₋ₓFₓ enthält, worin x null oder eine ganze Zahl von 1 bis 3 ist, und sowohl Cl₂ als auch HF, mit einer Katalysator-Zusammensetzung bei einer Temperatur im Bereich von 300 °C bis 450 °C, worin die Katalysator-Zusammensetzung aus einem Aluminiumfluorid hoher Reinheit besteht, das durch die Reaktion von Aluminiumhydroxid mit HF hergestellt wird, umfaßt.

8. Verfahren gemäß Anspruch 7, worin der Katalysator durch Ausflösen von Aluminiumhydroxid in wäßrigem HF, Eindampfen der erhaltenen Lösung, um einen Rückstand eines Aluminiumfluorids zu erhalten, und Erwärmen des Rückstandes unter Bildung eines getrockneten Feststoffs hergestellt wird.

9. Verfahren gemäß Anspruch 7, worin das Aluminiumhydroxid durch die Hydrolyse von AlR₃ oder die Hydrolyse von Al(OR)₃, worin jedes R unabhängig voneinander aus C₁- bis C₆-Alkylgruppen ausgewählt ist, hergestellt wird.

## Revendications (Revendications pour l'(les) Etat(s) contractant(s) suivant(s): AT, BE, CH, DE, FR, GB, IT, LI, LU, NL, SE)

1. Procédé de préparation de 1,1-dichloro-1,2,2,2-tétrafluoroéthane par chlorofluoration comprenant l'étape de mise en contact d'un mélange gazeux contenant au moins un tétrahalogénoéthylène de formule C₂Cl₄₋ₓFₓ, où x est un entier de 0 à 3, ainsi que du Cl₂ et du HF avec un catalyseur à une température comprise dans la gamme de 300°C à 450°C, ledit catalyseur étant constitué de fluorure d'aluminium de pureté élevée préparé en faisant réagir de l'hydroxyde d'aluminium avec du HF.

2. Procédé selon la revendication 1, dans lequel ledit tétrahalogénoéthylène est le CCl₂-CCl₂.

3. Procédé selon la revendication 1 dans lequel les sous-produits ayant moins de cinq atomes de fluor par molécule sont recyclés.

4. Procédé selon la revendication 1 dans lequel le rapport molaire dudit HF au dit tétrahalogénoétnylène est compris dans une gamme de 1:1 à 20:1 ; et dans lequel le rapport molaire dudit Cl₂ au dit tétrahalogénoéthylène est compris dans une gamme de 1:1 à 2:1.

5. Procédé selon la revendication 1 dans lequel les temps de contact sont compris dans la gamme de 5 à 100 secondes.

6. Procédé selon la revendication 1, dans lequel le catalyseur est pré-traité avec du gaz HF avant qu'il ne soit mis en contact avec ledit tétrahalogénoéthylène.

7. Procédé amélioré de fabrication du 2-chloro-1,1,1,2-tétraflluoroéthane et/ou du 1,1,1,2-tétrafluoroéthane par hydrodéchloration du 1,1-dichloro-1,2,2,2-tétrafluoroéthane, l'amélioration comprenant l'étape de préparation dudit 1,1-dichloro-1,2,2,2-tétrafluoroéthane par la mise en contact d'un mélange gazeux contenant au moins un tétrahalogénoéthylène de formule C₂Cl₄₋ₓFₓ, où x est zéro ou un entier de 1 à 3, ainsi que du Cl₂ et du HF avec un catalyseur à une température comprise dans la gamme de 300°C à 450°C, ledit catalyseur étant constitué de fluorure d'aluminium à pureté élevée préparé en faisant réagir de l'hydroxyde d'aluminium avec du HF.

8. Un catalyseur constitué de fluorure d 'aluminium à pureté élevée préparé en faisant réagir de l'hydroxyde d'aluminium avec du HF.

9. Le catalyseur selon la revendication 8 préparé en dissolvant de l'hydroxyde d'aluminium dans une solution aqueuse de HF ; évaporant la solution résultante pour obtenir un résidu de fluorure d'aluminium ; et chauffant ledit résidu pour produire un solide séché.

10. Le catalyseur selon la revendication 8 dans lequel ledit hydroxyde d'aluminium est préparé par l'hydrolyse de AlR₃ ou l'hydrolyse de Al(OR)₃ où chaque R est indépendamment choisi parmi les groupes alkyles ayant de 1 à 6 atomes de carbone.

## Revendications (Revendications pour l'(les) Etat(s) contractant(s) suivant(s): ES)

1. Procédé de préparation de 1,1-dichloro-1,2,2,2-tétrafluoroéthane par chlorofluoration comprenant l'étape de mise en contact d'un mélange gazeux contenant au moins un tétrahalogénoéthylène de formule C₂Cl₄₋ₓFₓ, où x est un entier de 0 à 3, ainsi que du Cl₂ et du HF avec un catalyseur à une température comprise dans la gamme de 300°C à 450°C, ledit catalyseur étant constitué de fluorure d'aluminium de pureté élevée préparé en faisant réagir de l'hydroxyde d'aluminium avec du HF.

2. Procédé selon la revendication 1, dans lequel ledit tétrahalogénoéthylène est le CCl₂-CCl₂.

3. Procédé selon la revendication 1 dans lequel les sous-produits ayant moins de cinq atomes de fluor par molécule sont recyclés.

4. Procédé selon la revendication 1 dans lequel le rapport molaire dudit HF au dit tétrahalogénoéthylène est compris dans une gamme de 1:1 à 20:1 ; et dans lequel le rapport molaire dudit Cl₂ au dit tétrahalogénoéthylène est compris dans une gamme de 1:1 à 2:1.

5. Procédé selon la revendication 1 dans lequel les temps de contact sont compris dans la gamme de 5 à 100 secondes.

6. Procédé selon la revendication 1, dans lequel le catalyseur est pré-traité avec du gaz HF avant qu'il ne soit mis en contact avec ledit tétrahalogénoéthylène.

7. Procédé amélioré de fabrication du 2-chloro-1,1,1,2-tétraflluoroéthane et/ou du 1,1,1,2-tétrafluoroéthane par hydrodéchloration du 1,1-dichloro-1,2,2,2-tétrafluoroéthane, l'amélioration comprenant l'étape de préparation dudit 1,1-dichloro-1,2,2,2-tétrafluoroéthane par la mise en contact d'un mélange gazeux contenant au moins un tétrahalogénoéthylène de formule C₂Cl₄₋ₓFₓ, où x est zéro ou un entier de 1 à 3, ainsi que du Cl₂ et du HF avec un catalyseur à une température comprise dans la gamme de 300°C à 450°C, ledit catalyseur étant constitué de fluorure d'aluminium à pureté élevée préparé en faisant réagir de l'hydroxyde d'aluminium avec du HF.

8. Procédé selon la revendication 7, dans lequel le catalyseur est préparé en dissolvant de l'hydroxyde d'aluminium dans une solution aqueuse de HF ; évaporant la solution résultante pour obtenir un résidu de fluorure d'aluminium ; et chauffant ledit résidu pour produire un solide séché.

9. Procédé selon la revendication 7, dans lequel ledit hydroxyde d'aluminium est préparé par l'hydrolyse de AlR₃ ou l'hydrolyse de Al(OR)₃ où chaque R est indépendamment choisi parmi les groupes alkyles ayant de 1 à 6 atomes de carbone.
